# EUROPÄISCHE PATENTSCHRIFT

(11) **EP 2 526 845 B1**
(45) Veröffentlichungstag und Bekanntmachung des Hinweises auf die Patenterteilung: **14.06.2017**
(21) Anmeldenummer: 12005841.7
(22) Anmeldetag: 16.12.2010
(51) Int. Cl.: A47K 3/28, A61N 5/06, A61H 33/06, A61H 33/00

(54) **Duscheinrichtung mit Wärmestrahlungsquelle**
Shower device with thermal radiation source
Dispositif de douche avec source de rayonnement thermique

(30) Priorität: 22.11.2010 AT 19292010; 21.01.2010 AT 4210 U
(43) Veröffentlichungstag der Anmeldung: 28.11.2012
(62) Teilanmeldung aus: 10450191.1
(73) Patentinhaber: NEHER GmbH, 9523 Villach-Landskron (AT)
(72) Erfinder: Weberndorfer, Birgit, 1090 Wien (AT); Tschudnig, Herbert, 9523 Villach-Landskron (AT); Ebner, Oliver, 9520 Annenheim (AT); Nachtmann, Markus, 5310 Mondsee (AT)
(74) Vertreter: Torggler & Hofinger Patentanwälte

(56) Entgegenhaltungen:
- WO-A1-2006/009358
- DE-A1- 3 317 140
- DE-A1-102007 032 585
- DE-A1-102008 056 587
- DE-B3- 10 313 031
- DE-U1- 20 104 556
- GB-A- 2 020 970

## Beschreibung

Die Erfindung betrifft eine Anordnung mit einer in Verbindung mit Wasser benützbaren Einrichtung in Kombination mit wenigstens einer in den Bereich der mit Wasser benützbaren Einrichtung Wärmestrahlen abgebenden Quelle, wobei die Wärmestrahlen abgebende Quelle eine Quelle für Infrarot-Strahlen ist, wobei die Quelle in einem Raum, der an wenigstens eine Wand der mit Wasser benützbaren Einrichtung angrenzt, vorgesehen ist.

Gattungsgemäße Einrichtungen werden beispielsweise in der DE 33 17 140 A1, der WO 2006 009358A1, DE 10 2008 056587A1, der DE 103 13 031 B3 und der DE 201 04 556 U1 gezeigt. Solche Einrichtungen können z.B. sein, Duschen, beispielsweise in Form von Duschkabinen oder freistehenden Duschen (Brausen), Badewannen, Whirlpools, Dampfduschen, Schwimmbäder. Solche Einrichtungen, die im Rahmen der Erfindung in Betracht gezogen sind, sind Einrichtungen, in denen Wasser im Zusammenhang mit dem menschlichen Körper angewendet wird, um auf den Körper beispielsweise eine reinigende oder eine für dessen Wohlbefinden verbessernde Wirkung auszuüben.

Weitere gattungsgemäße Einrichtungen zeigen die DE 10 2007 032585 A1 und die GB 2 020 970 A. Jedoch wird in diesen Schriften nicht gezeigt, dass die Infrarot-Strahlen abgebende Quelle relativ zu einem Fenster, welches sich in der Wand zwischen der Quelle und der mit Wasser benützbaren Einrichtung befindet, höhenverstellbar und/oder horizontal verstellbar und/oder in der Tiefe verstellbar ist.

Besonders in Betracht gezogen ist im Rahmen der Erfindung die Kombination einer Wärmestrahlen abgebenden Quelle (IR-Strahlen) mit einer Dusche beliebiger Bauart.

Unter dem hier verwendeten Begriff "Dusche" sind ganz allgemein Nassräume mit einer Duschvorrichtung ("Brausen") zu verstehen. Es handelt sich um Nassräume mit wenigstens einer Duschvorrichtung, die beispielsweise sein kann: eine Handbrause, eine an der Umgrenzung (Seitenwand und/oder Decke bzw. oberere Abdeckung) der Dusche gehaltene (gegebenenfalls auch abnehmbare) Brause, Düsen, die an wenigstens einer Umgrenzung (Wand, Duschabtrennung, Duschkabinendecke) vorgesehen sind oder Kombinationen derselben. Die Duschen, wie sie im vorliegenden Zusammenhang zu verstehen sind, können auch Badewannen mit Brause usw. sein, wobei gegebenenfalls eine (falt-, schwenk- oder schiebbare) Begrenzungswand vorgesehen sein kann.

Duschen können im vorliegenden Zusammenhang auch Brausen sein, die ohne jede seitliche Begrenzung frei angeordnet sind, also ohne seitliche Abgrenzungen oder Umgrenzungen, wie Türen, Seitenwände, obere Abdeckungen oder Ähnliches, ausgeführt sind.

Duschen sind in verschiedenen Ausführungen bekannt. So gibt es beispielsweise Duschen in Form von Duschkabinen, die frei stehend ausgebildet sind, ebenso wie Duschen, die wenigstens zum Teil in bestehende Bauwerke integriert sind. Häufig besitzen Duschen wenigstens eine seitliche Begrenzung, beispielsweise (zwei) Begrenzungswände, die entweder zur Gänze oder teilweise gebogen (rund) oder eben ausgebildet sind. Begrenzungen von Duschen können teilweise oder zur Gänze auch von bestehenden Wänden des Raumes, in dem die Dusche untergebracht ist, gebildet sein.

Ebenso sind Infrarot-Kabinen (Tiefenwärmekabinen) bekannt, in welchen der Körper des Benutzers von einer Infrarot-Quelle kommenden Infrarot-Strahlen ausgesetzt ist und durch diese beispielsweise erwärmt wird.

Oft ist es aus räumlichen oder anderen Gründen nicht möglich, zusätzlich zu einer in Verbindung mit Wasser benutzten Einrichtung, z.B. einer Dusche, eine Infrarot-Kabine aufzustellen.

Der Erfindung liegt die Aufgabe zugrunde, eine Anordnung vorzustellen, die zusätzlich zur Benutzung mit Wasser auch für die Wärmebehandlung, z.B. zur Infrarot-Therapie, benützt werden kann.

Gelöst wird diese Aufgabe erfindungsgemäß mit einer Anordnung, welche die Merkmale von Anspruch 1 aufweist.

Bevorzugte und vorteilhafte Ausgestaltungen der erfindungsgemäßen Anordnung sind Gegenstand der Unteransprüche.

Da bei der erfindungsgemäßen Anordnung eine Wärmequelle, die als Quelle für Infrarot-Strahlung ausgebildet ist, in Verbindung mit einer mit Wasser benützten Einrichtung, z.B. einer Dusche, kombiniert ist, besteht die Möglichkeit, die erfindungsgemäße Anordnung in verschiedenster Weise zu benützen. Nämlich nur die Einrichtung, die in Verbindung mit Wasser benützt wird (Dusche, Badewanne, u.dgl.) oder nur die Wärmequelle, den Infrarot-Strahler (ähnlich einer Infrarot-Kabine) alleine oder in Kombination beider Elemente der erfindungsgemäßen Anordnung, wobei die Möglichkeit besteht weitere Tätigkeiten auszuführen, beispielsweise indem die erfindungsgemäße Anordnung zusätzlich mit Einrichtungen für Schallabgabe (Musik, Audio) und Lichteffekte (z.B. LED-Licht) ausgestattet wird.

Insbesondere im Fall einer Dusche als in Verbindung mit Wasser benützbaren Einrichtung ergibt sich Folgendes:

Da bei der erfindungsgemäßen Dusche neben den üblichen Armaturen für eine Dusche, nämlich einer Brause und Ähnliches, auch wenigstens eine Quelle für Infrarot-Strahlung vorgesehen ist, besteht die Möglichkeit, die erfindungsgemäße Dusche wahlweise nur als Dusche oder nur für die Wärme-Therapie, insbesondere die Infrarot-Therapie, zu verwenden. Des Weiteren besteht die Möglichkeit, während des Duschens die Quelle für Wärme-Strahlung, insbesondere Infrarot-Strahlung, in Betrieb zu nehmen, so dass eine zusätzliche Erwärmung gegeben ist.

Die erfindungsgemäß vorgesehene, wenigstens eine Quelle für Infrarot-Strahlung, kann ein Infrarotstrahler, eine Infrarot-Strahlung abgebende Fläche, Platte oder dgl. sein. Wesentlich ist bloß, dass die Quelle für Wärme, insbesondere für Infrarot-Strahlung in den Bereich abstrahlt, in dem sich die Person befindet, welche die Einrichtung, die in Verbindung mit Wasser verwendet wird, der erfindungsgemäßen Anordnung, z.B. die Dusche, benützt.

Durch die erfindungsgemäße Anordnung wird beispielsweise eine platz- und kostensparende Kombination einer Infrarot-Kabine mit einer in Verbindung mit Wasser zu verwendenden Einrichtung, z.B. mit einer (Wellness)-Dusche, geschaffen. Mit der erfindungsgemäßen Anordnung, besteht auch die Möglichkeit, gleichzeitig Wasser zu verwenden, z.B. zu duschen und Tiefenwärme durch Infrarot-Strahlung zu empfangen, so dass bildlich davon gesprochen werden kann, dass die erfindungsgemäße Anordnung "Sonne und Regen" ins Bad holt.

Die erfindungsgemäße Anordnung hat auch den Vorteil, dass durch das Anwenden von Wasser, z.B. durch Benützen der Brause, eine Kühlung der Hautoberfläche herbeigeführt werden kann, wenn die Temperatur in der erfindungsgemäßen Anordnung zu hoch ist, oder aber ein Befeuchten des Körpers des Benützers, wenn es in der erfindungsgemäßen Anordnung zu trocken ist.

Zusätzlich besteht die Möglichkeit Wärme-Strahlung, die Infrarot-Strahlung, dazu zu benützen, den Körper der (sich beispielsweise duschenden) Person zusätzlich zu erwärmen, wenn der Person die Temperatur in der Anordnung, z.B. der Dusche, zu niedrig ist.

Schließlich besteht noch eine Möglichkeit, durch die Wärme die in der erfindungsgemäßen Anordnung entsteht, wenn die Quelle für Wärme-Strahlung (Infrarot-Strahlung) in Betrieb genommen ist, den Raum, z.B. ein Badezimmer, in dem sich die erfindungsgemäße Anordnung befindet, zu erwärmen. Hiebei kann vorgesehen sein, dass die in Verbindung mit Wasser verwendete Einrichtung, z.B. die Dusche (Duschkabine), nach oben hin offen ist, oder dass eine obere Abdeckung so ausgebildet ist, dass sie bei Bedarf geöffnet/geschlossen werden kann. Dieses Erwärmen des Raumes, in dem sich die erfindungsgemäße Anordnung befindet, durch die in der erfindungsgemäßen Anordnung vorgesehene Quelle für Wärme-Strahlung, insbesondere Infrarot-Strahlung, kann noch dadurch verstärkt werden, dass eine Zwangskonvektion durch Ventilatoren oder andere eine Luftbewegung erzeugende Einrichtungen herbeigeführt wird.

Für das Anordnen der wenigstens einen Quelle für Wärme, z.B. Infrarot-Strahlung, bestehen weitere, nicht erfindungsgemäße verschiedene Möglichkeiten. So kann die Wärme-Strahlung aus einem gesonderten, in der mit Wasser verwendeten Einrichtung (Dusche) aufgestellten, z.B. säulenartigen, Körper austreten, d.h. dass in diesem Fall die Quelle für Wärme-, z.B. Infrarot-Strahlung, z.B. der Infrarot-Strahler in einem, z.B. säulenartigen, in der Dusche oder im Bereich derselben aufgestellten Körper untergebracht ist.

Die wenigstens eine Quelle für Wärme-Strahlung (Infrarot-Strahlung) ist in einer Umgrenzung, in wenigstens einer Wand, welche die mit Wasser zu benützende Einrichtung, z.B. die Dusche, auf wenigstens einer Seite begrenzt, untergebracht. Dabei besteht die Möglichkeit, eine Wärme-Quelle vorzusehen, oder aber mehrere Wärme-Quellen nebeneinander und/oder übereinander anzuordnen.

Die Wärme-Quelle, z.B. der Infrarot-Strahler, ist in einem Raum untergebracht, der durch wenigstens ein "Fenster" mit dem Innenraum der mit Wasser zu verwendenden Einrichtung, z.B. der Dusche, in Verbindung steht. Dieser Raum ist in eine seitliche Wand der Dusche (Duschkabine) integriert. Das Fenster ist für Infrarot-Strahlung durchlässig und besteht bevorzugt aus Glas, insbesondere aus Einscheibensicherheitsglas ("ESG") oder aus Verbundsicherheitsglas ("VSG").

Der Raum, in welchem die Quelle für Infrarot-Strahlung untergebracht ist, kann durch eine Türe zugänglich sein, oder aber es ist die Möglichkeit gewählt, die Quelle für Wärme, insbesondere für Infrarot-Strahlung, und die zugehörigen Steuerungselemente an einem Rahmen zu montieren, der (nach Art eines Apothekerauszuges) aus dem Raum herausgezogen werden kann, um Montage-, Wartungs-, Einstell- oder Reparaturarbeiten vornehmen zu können. Für das Ein- und Ausfahren des Rahmens können motorische Antriebe vorgesehen sein.

Bei der erfindungsgemäßen Anordnung kann im Bereich des Fensters, durch das die Infrarotstrahlen, in die in Verbindung mit Wasser verwendete Einrichtung, z.B. in die Duschkabine, eintritt, insbesondere unterhalb des Fensters, ein Sitz für einen Benutzer vorgesehen sein. Dieser Sitz kann abklappbar sein, damit er beim normalen Benützen der Einrichtung (Dusche) nicht stört.

Um die Höhe des Sitzes an die Größe der ihn benützenden Person anzupassen, kann er höhenverstellbar sein. Diese Höhenverstellbarkeit kann stufenlos sein oder es sind bestimmte Höhenstellungen vorgegeben. Das Verstellen des Sitzes kann von Hand oder durch einen ihm zugeordneten Antrieb (elektrisch, pneumatisch, hydraulisch) erfolgen, wobei auch in Betracht gezogen ist, bei einem motorischen Antrieb für die Höhenverstellung des Sitzes bestimmte personenbezogene Höhenlagen zu speichern, sodass die jeweils gewünschte Höhenausrichtung des Sitzes wählbar ist, wenn die Person beabsichtigt die erfindungsgemäßen Anordnung zu benützen.

Wenn der Sitz höhenverstellbar ist, ist aber auch unabhängig davon, die in der Anordnung der Erfindung vorgesehene Wärmequelle, als Infrarot-Strahler ausgebildet, höhenverstellbar. Durch diese Höhenverstellbarkeit kann der Bereich, in den Wärmestrahlen, insbesondere Infrarot-Strahlen, abgegeben werden, an die Größe und/oder die Stellung (stehend/sitzend) der die erfindungsgemäße Anordnung benützenden Person angepasst werden. Für das Verstellen des Infrarot-Strahlers in Höhenrichtung, also auf und ab, kann ein elektrischer, pneumatischer oder hydraulischer Antrieb vorgesehen sein, also ein motorischer Antrieb oder aber der Infrarot-Strahler wird durch eine Handkurbel oder Ähnliches von Hand aus in die gewünschte Höhenlage verstellt.

Bei einer motorischen Verstellung der Höhenausrichtung der Quelle für Wärme, insbesondere für Infrarot-Strahler, kann auch vorgesehen sein, dass bestimmte Höhenausrichtungen in einem Speicher abgespeichert werden und so dass bei Bedarf durch Anwählen einer gespeicherten Stellung die Quelle für Wärme-Strahlung (insbesondere Infrarot-Strahlung) automatisch in die jeweils erforderliche Höhenausrichtung verstellt wird.

Wenn die Quelle für Wärmestrahlung in der erfindungsgemäßen Anordnung höhenverstellbar ist, kann ihr eine Blende zugeordnet sein, die mit ihr höhenverstellbar ist und verhindert, dass die "Technik" im Bereich der Quelle für Wärmestrahlung durch das Fenster, durch das die Wärmestrahlung in die Einrichtung, die in Verbindung mit Wasser benützt wird (z.B. einer Dusche), eintritt, sichtbar ist.

In einer Ausführungsform der Erfindung sind, insbesondere wenn ein Sitz vorgesehen ist, seitlich des Fensters für den Eintritt von Infrarotstrahlen, in die in Verbindung mit Wasser benutze Einrichtung, z.B. die Duschkabine, Rückenpolster vorgesehen, die verhindern, dass das Fenster, insbesondere das Infrarotfenster, z.B. mit dem Rücken, unbeabsichtigt berührt wird. Diese Rückenpolster können höhenverstellbar sein, damit sie in die für ein Benützen im Sitzen und ein Benützen im Stehen richtige Höhenlage gebracht werden können. Diese Verstellbarkeit kann beispielsweise dadurch gegeben sein, dass die Rückenpolster in unterschiedlicher Höhenlagen an Kupplungsvorsprünge rechts und links des Infrarotfensters eingehängt werden können.

In einer Ausführungsform der Erfindung ist auch vorgesehen, dass das Fenster, durch das die Infrarotstrahlen, in die Einrichtung abgibt, vertieft angeordnet ist, also in einer Nische liegt, sodass hier ein größerer Abstand vorliegt, der ein unbeabsichtigtes Berühren des Infrarotfensters (dieses kann warm sein) verhindert. Wenn zusätzlich ein Sitz (siehe oben) vorgesehen ist, kann dieser so angeordnet sein, dass er in der abgeklappten Stellung in der Vertiefung (Nische) aufgenommen ist, also in seiner abgeklappten Stellung nicht in den Innenraum der Einrichtung (z.B. der Dusche) vorsteht.

Wenn in der in Verbindung mit Wasser betriebenen Einrichtung, z.B. eine Dusche, in einer Wand eine Vertiefung (Nische) vorgesehen ist, kann in der Nische beispielsweise oberhalb des Fensters, durch das Wärmestrahlung (Infrarot-Strahlen) in die Einrichtung gelangen, eine Ablage für im Zusammenhang mit dem Benützen der Einrichtung verwendbare Mittel oder Geräte, beispielsweise in Form einer in der Nische vorgesehenen Platte (Glasplatte), vorgesehen sein.

Die Leistung der Infrarotstrahlenquelle (IR-Strahler), kann regelbar sein, wobei eine stufenweise oder eine stufenlose Regelung in Betracht gezogen ist. Dabei ist auch in Betracht gezogen, die Leistung der Wärmequelle durch einen Temperaturfühler, der in der in Verbindung mit Wasser benützten Einrichtung angeordnet ist, zu regeln.

Die erfindungsgemäß in der Anordnung vorgesehene Einrichtung, z.B. eine Dusche, kann auch mit wenigstens einer Lichtquelle, insbesondere in Form von LEDs, ausgestattet sein, die so angesteuert werden können, dass sie nach einem vorgegebenen oder wählbaren Programm Licht mit unterschiedlicher Farbe abgeben. So kann die erfindungsgemäße Anordnung auch mit wenigstens einer Lichtquelle (insbesondere Leds, die Licht mit verschiedenen Farben abgeben) ausgestattet werden. Diese Lichtquelle kann im Bereich neben dem Fenster, durch das Wärmestrahlen treten, vorgesehen sein. Bevorzugt sind Lichtquellen auf beiden Seiten des Fensters vorgesehen.

Weiters besteht im Rahmen der Erfindung die Möglichkeit, in der erfindungsgemäßen Anordnung Schallquellen (Lautsprecher) vorzusehen, sodass gegebenenfalls zusätzlich zur Lichttherapie auch eine Schalltherapie, beispielsweise durch entspannende Musik, möglich ist.

Wenn wenigstens eine Schallquelle (Lautsprecher) vorgesehen ist, kann diese dem Fenster, durch das Infrarot-Strahlen treten, nach Art eines "Körperschall-Lautsprechers" zugeordnet sein, so dass das Fenster (insbesondere die Fensterscheibe) als Resonanzkörper dient, sodass Schall - gleich welcher Art (vorzugsweise Musik) - besonders gut in den Raum der mit Wasser benützten Einrichtung tritt. Die Verwendung eines sog. Körperschallwandlers ist im Rahmen der Erfindung auch deshalb von Vorteil, da dieser als unsichtbarer Lautsprecher vor dem in der Einrichtung, insbesondere Dusche, verwendetem Wasser geschützt ist.

Wenn ein Körperschallwandler ohne Lautsprechermembran, umfassend eine schwingfähige Masse, wenigstens einen Anschluss zu einem elektronischen Bauteil, insbesondere zur elektrischen Verbindung mit einem Verstärker, und eine Montageplatte, als Schallquelle vorgesehen ist, ist es bevorzugt, wenn die Montageplatte auf dem Fenster für den Durchtritt von Wärmestrahlen angeordnet ist. Durch das Anlegen eines Signals, insbesondere eines Musiksignals, vibriert die schwingfähige Masse. Die Schwingung überträgt sich auf die Montageplatte und wird von dieser in Form von Biegewellen an das Fenster für den Durchtritt von Wärmestrahlen als anzuregende Platte weitergegeben. Das Fenster strahlt dann das Signal in die Einrichtung, insbesondere die Dusche, ab.

Wenn bei der erfindungsgemäßen Einrichtung das "Fenster", durch das die Infrarot-Strahlen treten, in einer Nische angeordnet ist, können Lautsprecher, bevorzugt an den Scheiben links und rechts neben der Nische, angeordnet sein.

Das Anordnen der Montageplatte am Fenster kann form- und/oder kraftschlüssig erfolgen, beispielsweise durch schrauben und/oder kleben.

Das Anordnen des Körperschallwandlers erfolgt insbesondere außermittig am Fenster, da so einerseits die Signalwiedergabe besonders gut stattfindet und andererseits sich kein Bauteil vor der Wärmestrahlen abgebende Quelle, insbesondere ein Infrarot-Strahler, befindet.

Im Rahmen der Erfindung ist auch in Betracht gezogen, mit der erfindungsgemäßen Anordnung gleichzeitig wenigstens zwei der nachstehend genannten Verwendungsmöglichkeiten vorzunehmen: Thermaltherapie, Lichttherapie, Anwenden von Wasser (duschen, baden u. dgl.) und Einwirken von Wärme-Strahlung, insbesondere Infrarot-Strahlung.

In der in Verbindung mit Wasser benützbaren Einrichtung, insbesondere in einer Dusche, die bei der erfindungsgemäßen Anordnung mit der Quelle für Wärmestrahlen kombiniert ist, können im Rahmen der Erfindung Geräte für das Ausüben von Gymnastik oder sonstigen der körperlichen Ertüchtigung dienenden Übungen, vorgesehen sein. Beispielsweise können Einrichtungen für das Strecken, Dehnen und Kräftigen vorgesehen sein.

Wenn in der Einrichtung solche Geräte vorgesehen sind, besteht im Rahmen der Erfindung die Möglichkeit, in der der Wärme-Quelle zugeordneten in Verbindung mit Wasser benützbaren Einrichtung der erfindungsgemäßen Anordnung, Anweisungen für das Benützen dieser Einrichtungen, beispielsweise in Form von Bildern (Piktogramme) und/oder verbalen Beschreibungen der Übungen anzubringen. Zusätzlich besteht bei der Ausführungsform, bei welcher in der erfindungsgemäßen Anordnung eine Schallquelle vorgesehen ist, die Möglichkeit Anweisungen der Benützung der Geräte zur Körperertüchtigung oder Gymnastik auch über diese Schallquelle abzugeben.

In der Einrichtung, die in Kombination mit einer Wärmequelle in der erfindungsgemäßen Anordnung vorgesehen ist, können für die Verwendung von Wasser auch Wassernebeldüsen neben anderen Düsen (Brausen) oder nur Wassernebeldüsen vorgesehen sein. Diese Wassernebeldüsen haben insbesondere dann einen erfrischenden/abkühlenden Effekt, wenn die erfindungsgemäße Anordnung ausschließlich zur Wärmebehandlung eingesetzt wird.

In einer Ausführungsform der erfindungsgemäßen Anordnung kann die Wärmequelle, insbesondere der Infrarot-Strahler, in einer Baueinheit (Box) vorgesehen sein, die beliebig aufstellbar sind und Einrichtungen, die in Verbindung mit Wasser verwendet werden (beispielsweise seitliche Begrenzung einer Dusche), zugeordnet werden können. In diesen Baueinheiten können zusätzlich zu der Wärmequelle (Infrarot-Strahler) auch andere Einrichtungen, wie eine Installation für die Zufuhr von Wasser zu einer Brause oder anderen Wasser abgebenden Düsen (Wassernebeldüsen) mit den zugehörigen Armaturen vorgesehen sein. Des Weiteren besteht die Möglichkeit in dieser Baueinheit (Box) auch Vorrichtungen zur Wasseraufbereitung (Reinigen, Filtern, Enthärten) und/oder zur Wassererwärmung (Boiler) untergebracht sein.

In einer Ausführungsform der erfindungsgemäßen Anordnung ist in Betracht gezogen, die Trennwand zwischen der Wärmequelle und der Einrichtung, die mit Wasser verwendet wird, aus durchsichtigem Werkstoff, beispielsweise Glasbausteinen, herzustellen.

Wenn die Wärmestrahlen abgebende Einrichtung eine Infrarot-Strahlenquelle ist, gibt diese Quelle in der Regel Strahlung mit einer Wellenlänge von 600 - 2200 nm ab. Dabei ist der Bereich oberhalb einer Wellenlänge von 1400 nm (IR-B- und IR-C-Strahlung) unerwünscht, da er eine Tiefenwärmewirkung mit thermischer Oberflächenbelastung der Haut entfaltet. Es ist daher ein Vorteil der erfindungsgemäßen Anordnung, dass die Quelle für Infrarot-Strahlung ein Strahler mit Infrarot-A Anteil Verwendung findet und dieser in Kombination mit Wasser in der zugeordneten Einrichtung verwendet wird, da Wasser die unerwünschten IR-B- und IR-C-Strahlungsanteile herausfiltert.

Dieser Effekt des Herausfilterns der IR-B- und IR-C-Strahlungsanteile kann noch verstärkt werden, indem dem Fenster, durch das die IR-Strahlung der zugeordneten Einrichtung, die in Verbindung mit Wasser verwendet wird, gelangt, eine Einrichtung zum Erzeugen eines Wasserfilms, beispielsweise in Form einer flachen oder langgestreckten Düse (oder eine Mehrfachdüse), zugeordnet wird. Durch diesen Wasserfilm im Bereich des Fensters, durch das Infrarot-Strahlung tritt, wird der IR-B-und IR-C-Strahlungsanteil wirksam herausgefiltert und tritt nicht in die der Infrarot-Strahlenquelle zugeordnete Einrichtung.

Bevorzugte Ausführungsformen der erfindungsgemäßen Anordnung können wie folgt zusammengefasst werden:

Die Erfindung zeichnet sich unter anderem dadurch aus, dass die Quelle für Wärme eine Quelle für Infrarot-Strahlung ist.

In einer Ausführungsform kann sich die Erfindung dadurch auszeichnen, dass die in Verbindung mit Wasser benützbare Einrichtung eine Dusche, ein Schwimmbad, ein Whirlpool, eine Dampfdusche und/oder eine Badewanne ist.

Es ist vorgesehen, dass zwischen der Wärmestrahlen abgebenden Quelle einerseits und der in Verbindung mit Wasser verwendbaren Einrichtung andererseits ein Fenster für den Durchtritt von Infrarot-Strahlen, ist.

In einer Ausführungsform kann sich die Erfindung dadurch auszeichnen, dass dem Fenster für den Durchtritt von Infrarot-Strahlen, eine für Infrarot-Strahlen durchlässige Abdeckung zugeordnet ist.

In einer Ausführungsform kann sich die Erfindung dadurch auszeichnen, dass der Abdeckung des Fensters für den Durchtritt von Infrarot-Strahlen, eine Einrichtung zum Erzeugen eines Wasserfilms zugeordnet ist.

In einer Ausführungsform kann sich die Erfindung dadurch auszeichnen, dass die Einrichtung eine Flachdüse, die im Bereich des oberen Randes der Öffnung vorgesehen und mit Wasser beaufschlagbar ist, ist.

In einer Ausführungsform kann sich die Erfindung dadurch auszeichnen, dass die in Verbindung mit Wasser verwendbare Einrichtung eine Dusche ist, der eine Quelle für Infrarot-Strahlung zugeordnet ist.

In einer Ausführungsform kann sich die Erfindung dadurch auszeichnen, dass die Quelle für Infrarot-Strahlung in wenigstens einem, die Dusche begrenzenden, Bauteil vorgesehen ist.

In einer Ausführungsform kann sich die Erfindung dadurch auszeichnen, dass die wenigstens eine Quelle für Infrarot-Strahlung in wenigstens einer Wand der Dusche vorgesehen ist.

Die Erfindung zeichnet sich zudem dadurch aus, dass die Quelle für Infrarot-Strahlung ein Infrarot-Strahler ist.

In einer Ausführungsform kann sich die Erfindung dadurch auszeichnen, dass der Infrarot-Strahler als strahlende Fläche oder Platte ausgebildet ist.

In einer Ausführungsform kann sich die Erfindung dadurch auszeichnen, dass die Quelle für Infrarot-Strahlung in wenigstens einer seitlichen Wand der Duschkabine vorgesehen ist.

In einer Ausführungsform kann sich die Erfindung dadurch auszeichnen, dass in wenigstens einer Wand der Duschkabine mehr als eine Quelle für Infrarot-Strahlung vorgesehen ist.

In einer Ausführungsform kann sich die Erfindung dadurch auszeichnen, dass die Quelle für Infrarot-Strahlung in einem Raum, der an wenigstens eine Wand der Duschkabine angrenzt, vorgesehen ist.

In einer Ausführungsform kann sich die Erfindung dadurch auszeichnen, dass der Raum durch eine (Schwenk-)Türe zugänglich ist.

In einer Ausführungsform kann sich die Erfindung dadurch auszeichnen, dass die Quelle für Infrarot-Strahlung in dem Raum an einem, vorzugsweise mit Hilfe eines Antriebes, herausfahrbaren Rahmen montiert ist.

In einer Ausführungsform kann sich die Erfindung dadurch auszeichnen, dass der Rahmen eine den Raum begrenzende Abdeckplatte trägt.

In einer Ausführungsform kann sich die Erfindung dadurch auszeichnen, dass in der Abdeckplatte Bedien- und Einstellorgane für die Quelle für Infrarot-Strahlung vorgesehen sind.

In einer Ausführungsform kann sich die Erfindung dadurch auszeichnen, dass die Abdeckplatte neben einer Duschtüre angeordnet ist.

In einer Ausführungsform kann sich die Erfindung dadurch auszeichnen, dass zwischen der Quelle für Infrarot-Strahlung und dem Innenraum der Duschkabine eine für Infrarot-Strahlung durchlässige Abdeckung vorgesehen ist.

In einer Ausführungsform kann sich die Erfindung dadurch auszeichnen, dass der Raum, in dem die wenigstens eine Quelle für Infrarot-Strahlung vorgesehen ist, in einer Wand, insbesondere in einer seitlichen Wand der Dusche vorgesehen ist.

In einer Ausführungsform kann sich die Erfindung dadurch auszeichnen, dass die Dusche nach oben hin offen ist.

In einer Ausführungsform kann sich die Erfindung dadurch auszeichnen, dass die Dusche oben durch eine Abdeckung verschlossen ist.

In einer Ausführungsform kann sich die Erfindung dadurch auszeichnen, dass die Abdeckung in eine offene und eine geschlossene Stellung verstellbar ist.

In einer Ausführungsform kann sich die Erfindung dadurch auszeichnen, dass in einer Seitenwand der Duschkabine ein Fenster für den Durchtritt von Infrarotstrahlung aus der Quelle für Infrarotstrahlung vorgesehen ist.

In einer Ausführungsform kann sich die Erfindung dadurch auszeichnen, dass rechts und links des Fensters für den Durchtritt von Infrarotstrahlung Rückenpolster vorgesehen sind.

In einer Ausführungsform kann sich die Erfindung dadurch auszeichnen, dass die Rückenpolster in wenigstens zwei unterschiedlichen Höhenlagen festlegbar sind.

In einer Ausführungsform kann sich die Erfindung dadurch auszeichnen, dass in der Duschkabine ein gegebenenfalls abklappbarer Sitz vorgesehen ist.

In einer Ausführungsform kann sich die Erfindung dadurch auszeichnen, dass der Sitz unterhalb des Fensters für den Durchtritt von Infrarotstrahlung aus der Quelle für Infrarot-strahlung vorgesehen ist.

In einer Ausführungsform kann sich die Erfindung dadurch auszeichnen, dass der in Verbindung mit Wasser verwendbaren Einrichtung, insbesondere der Duschkabine, wenigstens eine Lichtquelle, insbesondere LED, für Lichttherapie zugeordnet sind.

In einer Ausführungsform kann sich die Erfindung dadurch auszeichnen, dass der in Verbindung mit Wasser verwendbaren Einrichtung, insbesondere der Duschkabine, Schallquellen für Schalltherapie zugeordnet sind.

In einer Ausführungsform kann sich die Erfindung dadurch auszeichnen, dass die Schallquelle einen Körperschallwandler umfasst.

In einer Ausführungsform kann sich die Erfindung dadurch auszeichnen, dass der Körperschallwandler eine schwingfähige Masse, wenigstens einen Anschluss zu einem elektronischen Bauteil, insbesondere zur elektrischen Verbindung mit einem Verstärker, und eine Montageplatte umfasst, wobei die Montageplatte am Fenster für den Durchtritt von Wärmestrahlen, insbesondere von Infrarot-Strahlen, angeordnet, insbesondere außermittig angeordnet, ist.

In einer Ausführungsform kann sich die Erfindung dadurch auszeichnen, dass das Fenster gegenüber der Wand der Duschkabine, neben welcher die Quelle für Infrarotstrahlung vorgesehen ist, als Nische vertieft ausgebildet ist.

In einer Ausführungsform kann sich die Erfindung dadurch auszeichnen, dass die Rückenpolster rechts und links der Nische vorgesehen sind.

In einer Ausführungsform kann sich die Erfindung dadurch auszeichnen, dass der Sitz in seiner abgeklappten Stellung in der Nische aufgenommen ist.

In einer Ausführungsform kann sich die Erfindung dadurch auszeichnen, dass die Leistung der Quelle für Infrarotstrahlung, einstellbar ist.

In einer Ausführungsform kann sich die Erfindung dadurch auszeichnen, dass die Leistung stufenweise oder kontinuierlich einstellbar ist.

In einer Ausführungsform kann sich die Erfindung dadurch auszeichnen, dass der in Verbindung mit Wasser benützbaren Einrichtung Vorrichtungen, z.B. Ventilatoren, zum Erzeugen von Konvektion in der Einrichtung zugeordnet sind.

In einer Ausführungsform kann sich die Erfindung dadurch auszeichnen, dass der die Wärmestrahlen abgebende Infrarot-Strahler und Installationen, wie Brausen, Düsen, Wasserleitungen und/oder Einrichtungen zum Aufbereiten und/oder Erwärmen von Wasser, für die mit Wasser betriebene Einrichtung und/oder elektronische Komponenten der Schallquelle, beispielsweise ein Verstärker, in einem vorgefertigten Bauteil, insbesondere einem Schrank, eingebaut sind, der der mit Wasser betriebenen Einrichtung zugeordnet ist.

Weitere Einzelheiten und Merkmale der Erfindung ergeben sich aus der nachstehenden Beschreibung bevorzugter Ausführungsbeispiele anhand schematischer Zeichnungen, in denen als Beispiel für eine erfindungsgemäße Anordnung, die mit einer Duschkabine in verschiedenen Ausführungsformen dargestellt ist.

Es zeigt:
Fig. 1: eine erfindungsgemäße Anordnung mit Dusche in Schrägansicht,
Fig. 2: eine andere Ausführungsform in Draufsicht,
Fig. 3: eine Seitenansicht der Anordnung von Fig. 2,
Fig. 4: in einem Diagramm die spektrale Strahlungsdichte eines Tiefenwärmestrahlers, der in der erfindungsgemäßen Anordnung verwendet ist,
Fig. 5: in einem Diagramm die spektrale Bestrahlungsstärke bei Verwendung eines Wasserfilms und
Fig. 6: in einem Diagramm die spektrale Bestrahlungsstärke beim Duschen.

Bevor im Einzelnen auf die Beschreibung der in den Zeichnungen dargestellten Ausführungsformen einer erfindungsgemäßen Anordnung mit einer Dusche eingegangen wird, ist darauf hinzuweisen, dass die Erfindung nicht auf Duschen, beispielsweise solche mit rechteckigem oder quadratischem Grundriss, beschränkt ist. Vielmehr kann die in einer erfindungsgemäßen Anordnung vorgesehene, in Verbindung mit Wasser benützbare Einrichtung, beispielsweise eine Dusche, eine beliebige Grundrissform aufweisen, wobei auch Umgrenzungen mit gebogenen Wänden oder Kombinationen aus gebogenen Wänden und geraden Wänden in Betracht gezogen sind. Insbesondere bei in einer Raumecke angeordneten Duschen besteht auch die Möglichkeit, die Tür der Duschkabine als um eine lotrechte Achse gewölbte Tür auszubilden. Wie erwähnt kann die Erfindung bei beliebig ausgebildeten Duschen (Duschvorrichtungen), wie Duschkabinen, Badewannen usw., verwirklicht sein.

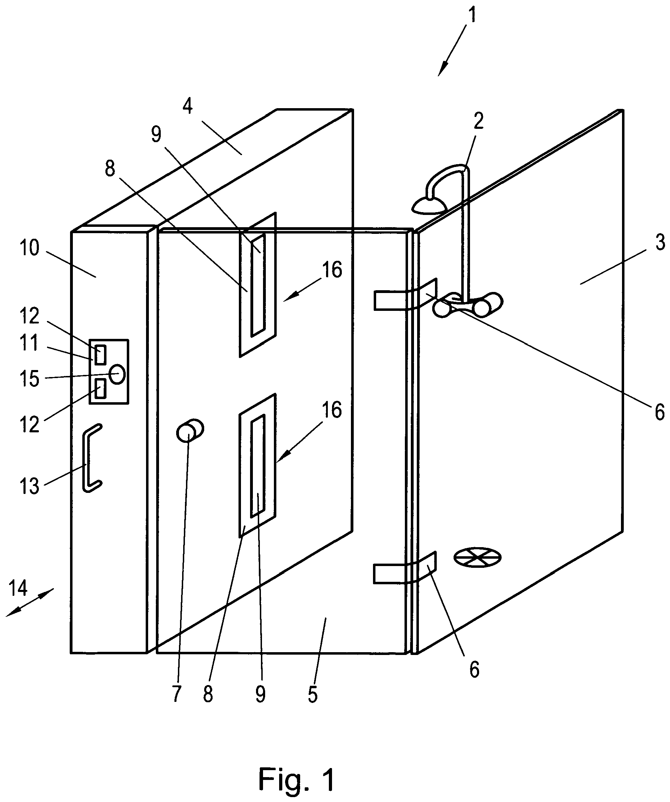

In der in Fig. 1 gezeigten beispielhaften Ausführungsform ist die in Verbindung mit Wasser benützbare Einrichtung 1 eine Dusche, die als Duschkabine ausgebildet ist. Die Duschkabine besitzt eine Rückwand, an der eine Duscharmatur 2 (Brause) mit dazugehörigen Regel-Armaturen montiert ist. Die Einrichtung 1 wird durch seitliche Wände 3, 4 begrenzt, wobei die rechts liegende seitliche Wand 3 im gezeigten Ausführungsbeispiel als Glaswand ausgebildet ist. An der seitlichen Wand 3 ist über Beschläge 6 eine schwenkbare Tür 5 angelenkt, die mit Hilfe eines Türgriffes 7 geöffnet und geschlossen werden kann. Die Tür 5 der Duschkabine kann auch eine Pendeltüre, Falttüre oder eine Schiebetüre sein. Es ist auch in Betracht gezogen, die Tür 5 an der Wand 4 anzuschlagen.

In der in Fig. 1 links liegenden seitlichen Wand 4 ist ein Raum untergebracht, in dem im gezeigten Ausführungsbeispiel als Wärmequelle zwei Quellen 9 übereinander untergebracht sind. In der der Duschkabine zugekehrten Seite der seitlichen Wand 4 sind zwei Fenster 16 vorgesehen, die mit einer Abdeckung 8 verschlossen sind, wobei die Abdeckung 8 für Infrarot-Strahlen durchlässig ist, aber verhindert, dass Spritzwasser beim Duschen auf die Quelle 9 trifft. Die Abdeckung 8 besteht beispielsweise aus Glas, wie VSG oder ESG.

Dem Fenster 16 bzw. der Abdeckung 8 kann in einer Ausführungsform im Bereich ihres oberen horizontalen Randes wenigstens eine Flachdüse zugeordnet sein, die mit Wasser beaufschlagt wird und auf der dem Innenraum der Duschkabine zugekehrten Seite einen Wasserfilm erzeugt. Dieser Wasserfilm hat den vorteilhaften Effekt, dass aus der von der Quelle 9 abgegebenen Strahlung die unerwünschten IR-B- und IR-C-Strahlungsanteile (Wellenlängen über 1400 nm) herausgefiltert werden und nur der besonders günstige und vorteilhafte Strahlungsbereich IR-A Strahlung von 800 - 1400 nm in den Innenraum der Duschkabine tritt.

Im gezeigten Ausführungsbeispiel wird davon ausgegangen, dass die Quelle 9 auf einem (nicht gezeigten) Rahmen montiert sind, der in dem in der seitlichen Wand 4 vorgesehenen Raum in Richtung des Doppelpfeiles 14 verschiebbar untergebracht ist. So kann der Rahmen mit seiner vorderen Abdeckplatte 10 mit Hilfe des Griffes 13 aus der Wand 4 herausgezogen werden, um Zugang zu den Infrarot-Strahlern 9 zu schaffen. Der Rahmen auf den die Quelle 9 aufgebaut sind können von Hand aus mit Hilfe des Griffes 13 aus der Wand 4 herausgezogen werden. Es besteht aber auch die Möglichkeit einen Antrieb, z.B. einen hydraulischen, pneumatischen oder elektromotorischen Antrieb, für das Herausfahren des Rahmens nach Art eines Apothekeraufzuges vorzusehen.

Der Raum unterhalb der Wand 4, in dem die Wärmestrahlen abgebende Quelle, z.B. wenigsten eine Quelle 9, aufgenommen bzw. untergebracht ist, kann durch eine Tür zugänglich sein.

Die Wand 4 kann, insbesondere auf ihrer von der mit Wasser betriebenen Einrichtung 1 abgekehrten Seite mit einer Ablage, in der Fächer vorgesehen sind und die gegebenenfalls mit wenigstens einer Tür verschlossen werden kann, ausgestattet sein. So können Utensilien, die in der mit Wasser betriebenen Einrichtung 1 verwendet werden, verstaut werden. So ist es nicht mehr nötig, diese Utensilien in der Duschkabine selbst bereitzuhalten.

In Fig. 1 ist noch gezeigt, dass an der Abdeckplatte 10, die den Raum nach vorne verschließt und die in ihrer Stellung, in welcher der Rahmen in den Raum in der Wand 4 eingeschoben ist, neben der Tür 5 angeordnet ist, ein Bedienfeld 11 mit Schaltern 12 und einer Kontrollleuchte 15 für die Steuerung der Quelle 9 vorgesehen ist.

In einer nicht gezeigten Alternative kann vorgesehen sein, dass der Raum in der seitlichen Wand 4 durch eine Schwenktür zugänglich ist, wobei die Schwenktür beispielsweise auf der den Fenstern 16 mit Abdeckung 8 gegenüberliegenden Seite der Wand 4 vorgesehen sein kann.

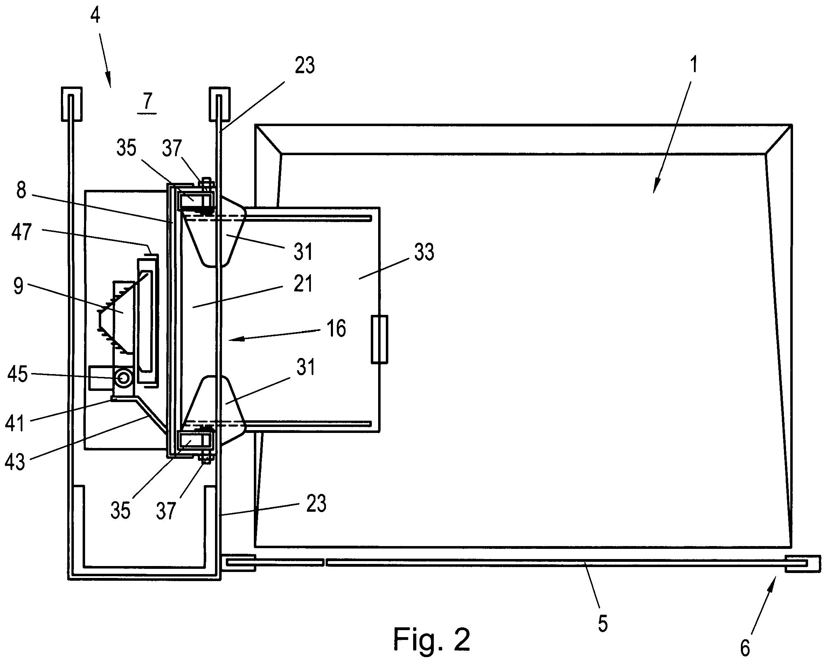

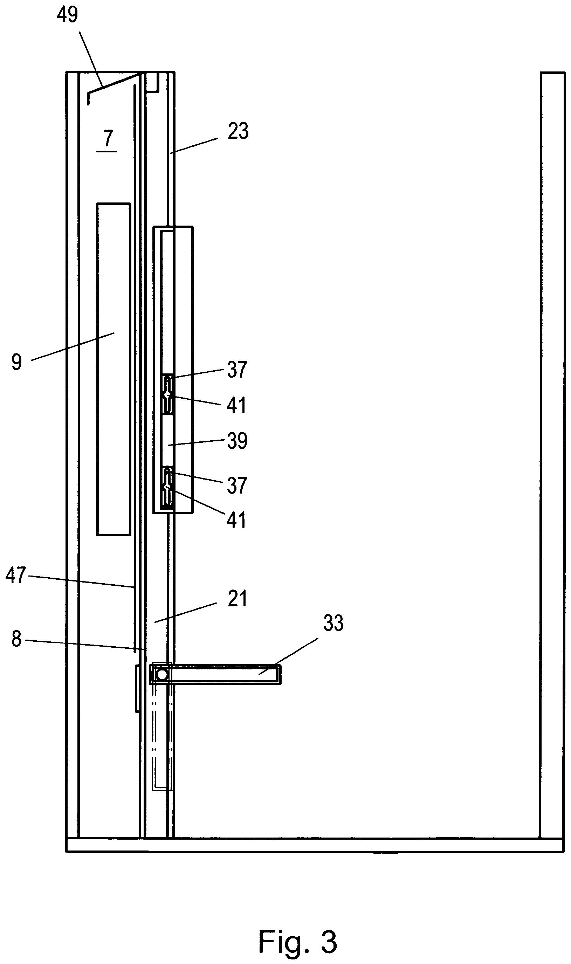

Bei der in den Figuren 2 und 3 gezeigten Ausführungsform einer erfindungsgemäßen Dusche in Form einer Duschkabine ist das Fenster 16 mit der Abdeckung 8 zwischen dem Innenraum der Einrichtung 1 und dem der Quelle 9 für Infrarot-Strahlung in einer Nische 21 vorgesehen. Dabei ist das Fenster 16 gegenüber der Wand 23 zu dem Raum 7, in welchem die Quelle 9 für Infrarot-Strahlung aufgenommen ist, in Richtung auf die Quelle 9 hin versetzt angeordnet.

Zu beiden Seiten des Fensters 16 für Infrarot-Strahlen sind in dem in den Figuren 2 und 3 gezeigten Ausführungsbeispiel Rückenpolster 31 vorgesehen, die verhindern, dass unbeabsichtigt der Rücken die Abdeckung 8 (z.B. aus Glas, insbesondere ESG oder VSG) im Fenster 16, durch die Infrarotstrahlen in den Innenraum der Einrichtung 1 treten, berührt.

Unterhalb des Fensters 16 für Infrarotstrahlung ist in dem in Fig. 2 und 3 gezeigten Ausführungsbeispiel ein abklappbarer Sitz 33 vorgesehen, der in der abgeklappten Stellung (vgl. Fig. 3) in der Vertiefung (Nische 21) aufgenommen ist, also in den Innenraum der Einrichtung 1 nicht vorsteht. Der Sitz 33 kann in einer bevorzugten Ausführungsform höhenverstellbar sein, wobei beispielsweise an den Seitenwänden der Nische 21 Führungen für den Sitz 33 vorgesehen sind. Das Verstellen des Sitzes 33 kann von Hand aus erfolgen, indem dieser in gewünschten Höhenlagen durch Einrasten von Arretierstiften in Löcher im Bereich der Seitenfläche der Nische 21 einrastet oder aber motorisch verstellbar sein (elektrischer, hydraulischer oder pneumatischer Antrieb), wobei im letzteren Fall auch die Möglichkeit besteht, bestimmte Höhenstellungen in einem Speicher abzulegen, sodass diese bei Bedarf abgerufen werden können. Das Höhenverstellen des Sitzes 33 erlaubt es, dessen Höhe an die Größe der die Einrichtung, nämlich die Einrichtung 1, benützenden Person, anzupassen.

Die Rückenpolster 31 sind in (wenigstens) zwei verschiedenen Höhenlagen festlegbar, sodass sie einmal eine Höhenausrichtung haben, die für das Benützen der Einrichtung 1 durch eine stehende Person und einmal für das Benützen der Einrichtung 1 durch eine Person, die auf dem aufgeklappten Sitz 33 sitzt, richtig ausgerichtet werden können. Hierzu sind in den Seitenteilen 35 der Nische 21 Aufnahmebolzen 37 vorgesehen. Die Rückenpolster 31 tragen auf ihrer der Wand zugekehrten Seite Metallschienen 39, in denen außermittig Führungen 41 vorgesehen sind, wie dies in Fig. 3 dargestellt ist. Durch Anheben der Rückenpolster 31 aus der in Fig. 3 gezeigten Lage bis die Aufnahmebolzen 37 in die breiteren Bereiche der beiden Führungen 41 - als doppelschlüssellochartige Öffnungen ausgebildet - gelangen, können die Rückpolster 31 abgenommen und nach Schwenken um 180° (das zunächst obere Ende gelangt nach unten und das vorher untere Ende nach oben) wieder eingehängt werden. So können die in Fig. 3 in die Stellung für eine stehende Person gezeigte Lage in eine Stellung für eine sitzende Person geändert werden, in welcher sie weiter nach unten ragen, da die als Führungen 41 dienenden doppelschlüssellochartigen Öffnungen in den Rückenpolstern 31 außermittig, also zum einen Ende hin versetzt, angeordnet sind.

Wie aus Fig. 2 erkennbar, ist die Quelle 9 für Infrarot-Strahlung (IR-Strahler) in dem Aufnahmeraum 7 für die Quelle 9 für Infrarot-Strahler so angeordnet, dass sie im Wesentlichen frei tragend aufgehängt ist. Dies erlaubt es auch, die Quelle 9 einfach an die jeweils gewünschte Höhe bezüglich des Durchtrittsfensters 16 für Infrarot-Strahlen auszurichten. Zusätzlich ist die Quelle 9 horizontal (seitlich) und/oder in der Tiefe (senkrecht zur Abdeckung 8 im Fenster 16) verstellbar. Die wenigstens eine Quelle 9 ist in dem Raum 7 der Wand 4 höhenverstellbar angeordnet, sodass seine Höhenausrichtung beispielsweise an die Höhe des Sitzes 33 angepasst werden kann oder aber die Höhe so eingestellt werden kann, dass die Ausrichtung des Infrarot-Strahlers 9 für eine sitzende bzw. eine stehende Person zutreffend ist.

Im Einzelnen ist die Quelle 9 an einer Führung 41, die an einem Ausleger 43 an der als Zwischenwand dienenden Wand 23 befestigt, angeordnet. Für die Höhenverstellbarkeit, die grundsätzlich manuell oder motorisch erfolgen kann, ist im gezeigten Ausführungsbeispiel ein Hebezylinder 45 vorgesehen. Dieser Zylinder 45 ist beispielsweise ein pneumatischer oder hydraulischer Zylinder. Ein elektromotorischer Antrieb für die Höhenverstellbarkeit ist ebenfalls in Betracht gezogen. Wenn ein motorischer Antrieb für die Höhenverstellbarkeit des Infrarot-Strahlers 9 vorgesehen ist, können ähnlich wie dies für den Sitz 33 vorgesehen ist, bestimmte Höhenausrichtungen in einem Speicher abgelegt und bei Bedarf abgerufen werden. Beispielsweise kann die Höhenverstellbarkeit des Infrarot-Strahlers 9 an die des Sitzes 33 gekoppelt sein, sodass beim Höhenverstellen des Sitzes 33 die Quelle 9 in die für eine sitzende Behandlung richtige Lage bewegt wird. Zusätzlich besteht die Möglichkeit die Höhenausrichtung des Infrarot-Strahlers 9 davon abhängig zu machen, ob der Sitz 33 in seine Wirklage aufgeklappt oder in seine Versorgungslage abgeklappt ist.

In den Fig. 2 und 3 ist noch gezeigt, dass der Quelle 9 eine Blende 47 zugeordnet ist, die verhindert, dass bei nach oben verschobener Quelle 9 durch den unteren Bereich des Sichtfensters 16 in die Technik im Raum 7 Einblick möglich ist.

Zusätzlich ist oberhalb des Infrarot-Strahlers 9, da der Raum 7 aus thermischen Gründen nach oben hin in der Regel offen sein wird, eine Abdeckung 49 vorgesehen, die das Eintreten von Spritzwasser verhindert.

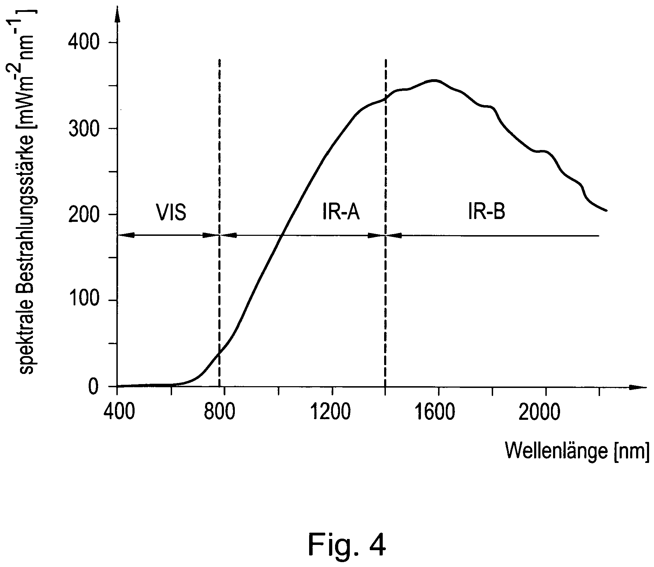

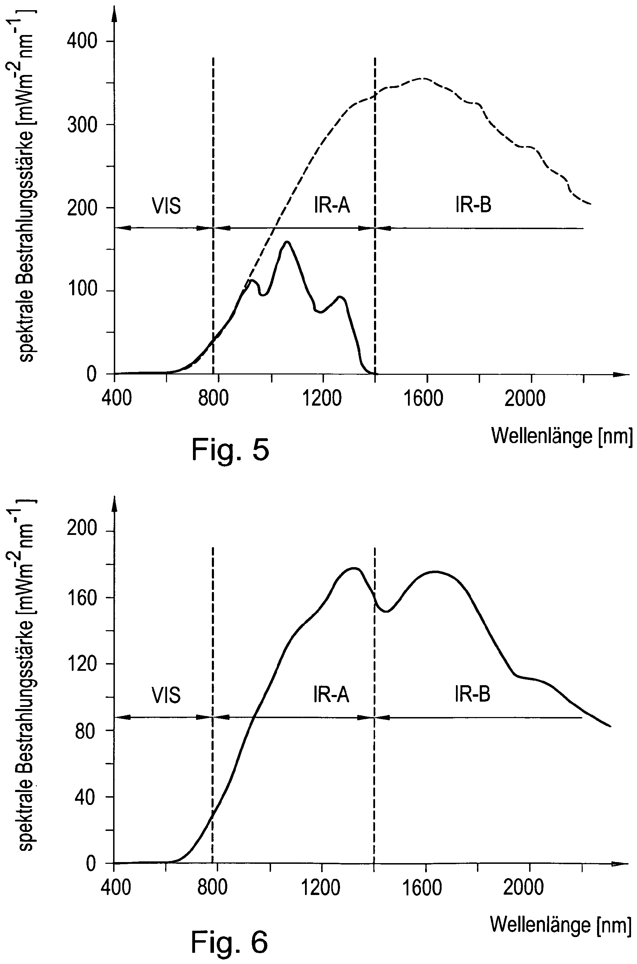

In den Figuren 4, 5 und 6 gezeigten Diagrammen sind die spektralen Strahlungsstärken eines erfindungsgemäß verwendbaren Infrarot-Strahlers 9 gezeigt (Fig. 4); Fig. 5 zeigt die spektrale Strahlungsstärke, wenn die vom Strahler 9 abgegebene Strahlung durch einen Wasserfilm tritt, Fig. 6 zeigt die spektrale Strahlungsstärke eines Infrarot-Strahlers 9, wenn dieser beim Benützen einer Einrichtung 1 in Betrieb genommen ist.

In einer nicht dargestellten Ausführungsform sind rechts und links des Fensters 16 für Infrarot-Strahlen, insbesondere außerhalb der beiden Bereiche, in welchen die Rückenpolster 31 angeordnet sind, Lichtquellen (beispielsweise LEDs) vorgesehen, die der Abgabe von Licht, insbesondere von Licht mit sich ändernder Farbe, geeignet sind, um eine Lichttherapie ausführen zu können.

In ähnlicher Weise können der Einrichtung 1 Lautsprecher (nicht gezeigt) zugeordnet sein, die der Abgabe von Schall, insbesondere Musik, zum Ausführen einer Schalltherapie verwendet werden können.

Fig. 2 zeigt, dass die Rückenpolster 31 so ausgebildet sind, das sie um die nach innen weisenden Kanten der Nische 21 greifen, also eine entsprechende Ausnehmung aufweisen. Dabei sind die Metallschienen 39 mit den Führungen 41 an den Rückenpolstern 31 so angeordnet, dass die Aufnahmebolzen 37 mittels welcher die Rückenpolster 31 an die Wand 23 gehängt werden können, an den Schmalflächen der Nische 21, also rechts und links des Fensters 16 aufeinander zuweisend, vorgesehen sein können.

Die Leistung der Infrarotquelle 9 (Infrarotstrahler) kann einstellbar sein, wobei eine stufenweise oder eine kontinuierliche Verstellung, gegebenenfalls in Kombination mit einem Thermostat, in Betracht gezogen ist.

Die in der erfindungsgemäßen Anordnung vorgesehene Wärmequelle, die beispielsweise in einem vorgefertigten Bauteil (Box) gegebenenfalls zusammen mit anderen Einrichtungen, wie Wasserversorgung, einer Brause, Düsen und Ähnliches kombiniert ist, kann auch als Baueinheit zum Nachrüsten bestehender in Verbindung mit Wasser benützbarer Einrichtungen, wie Duschen u. dgl., ausgebildet sein.

Zusammenfassend kann ein Ausführungsbeispiel der Erfindung wie folgt beschrieben werden:

Eine Anordnung umfasst eine Duschkabine mit Duscharmatur 2 und eine in einer seitlichen Wand 4 angeordneten Quelle 9 für Infrarot-Strahlung. In der Wand 4 sind Fenster 8 vorgesehen, die für Infrarot-Strahlung durchlässig sind. So kann die Duschkabine zum Duschen und/oder zum Einwirken lassen von Infrarot-Strahlung benützt werden. In der Duschkabine ist in einer Nische 21 in der auch das Fenster 16 angeordnet ist, ein Sitz 33 vorgesehen, wobei seitlich des Fensters 16 Rückenpolster 31 höhenverstellbar angeordnet sind.

## Patentansprüche

1. Anordnung mit einer in Verbindung mit Wasser benützbaren Einrichtung (1) in Kombination mit wenigstens einer in den Bereich der mit Wasser benützbaren Einrichtung (1) Wärmestrahlen abgebenden Quelle (9), wobei die Wärmestrahlen abgebende Quelle eine Quelle (9) für Infrarot-Strahlen ist, wobei die Quelle (9) in einem Raum, der an wenigstens eine Wand (23) der mit Wasser benützbaren Einrichtung (1) angrenzt, vorgesehen ist,
**dadurch gekennzeichnet, dass** die Infrarot-Strahlen abgebende Quelle (9) relativ zu einem Fenster (16), welches sich in der Wand (23) zwischen der Quelle (9) und der mit Wasser benützbaren Einrichtung (1) befindet, höhenverstellbar und/oder horizontal verstellbar und/oder in der Tiefe verstellbar ist.

2. Anordnung nach Anspruch 1, **dadurch gekennzeichnet, dass** die Quelle (9) für Infrarot-Strahlen ein Infrarot-Strahler ist.

3. Anordnung nach Anspruch 1 oder 2, **dadurch gekennzeichnet, dass** die in Verbindung mit Wasser benützbare Einrichtung (1) eine Duschkabine, ein Schwimmbad, ein Whirlpool, eine Dampfdusche und/oder eine Badewanne ist.

4. Anordnung nach einem der Ansprüche 1 bis 3, **dadurch gekennzeichnet, dass** dem Fenster (16) für den Durchtritt von Infrarot-Strahlen eine für Infrarot-Strahlen durchlässige Abdeckung (8) zugeordnet ist und

5. Anordnung nach Anspruch 4, **dadurch gekennzeichnet, dass** der Abdeckung (8) des Fensters (16) für den Durchtritt von Wärmestrahlen, insbesondere Infrarot-Strahlen, eine Einrichtung zum Erzeugen eines Wasserfilms zugeordnet ist.

6. Anordnung nach einem der Ansprüche 1 bis 5, **dadurch gekennzeichnet, dass** die in Verbindung mit Wasser betriebene Einrichtung (1), insbesondere Dusche, nach oben hin offen ist.

7. Anordnung nach einem der Ansprüche 1 bis 6, **dadurch gekennzeichnet, dass** der in Verbindung mit Wasser verwendbaren Einrichtung (1), insbesondere Dusche, wenigstens eine Lichtquelle, insbesondere LED, für Lichttherapie, und/oder wenigstens eine Schallquelle, insbesondere Musik und/oder Audio, für Schalltherapie, zugeordnet sind.

8. Anordnung nach Anspruch 7, **dadurch gekennzeichnet, dass** die Schallquelle einen Körperschallwandler umfasst, der im Bereich des Fensters (16), insbesondere seitlich neben dem Fenster (16) für den Durchtritt von Wärmestrahlen, insbesondere von Infrarot-Strahlen, angeordnet ist.

9. Anordnung nach einem der Ansprüche 7 bis 8, **dadurch gekennzeichnet, dass** der Infrarot-Strahler und Installationen, wie Brausen, Düsen, Wasserleitungen und/oder Einrichtungen zum Aufbereiten und/oder Erwärmen von Wasser, für die mit Wasser betriebene Einrichtung (1) und/oder elektronische Komponenten der Schallquelle in einem vorgefertigten Bauteil, insbesondere einem Schrank, eingebaut sind, der der mit Wasser betriebenen Einrichtung (1) zugeordnet ist.

10. Anordnung nach Anspruch 9, **dadurch gekennzeichnet, dass** in dem Bauteil, insbesondere dem Schrank, wenigstens eine Ablage für Badeutensilien vorgesehen ist.

11. Anordnung nach Anspruch 9 oder 10, **dadurch gekennzeichnet, dass** der Bauteil, insbesondere der Schrank, wenigstens teilweise aus durchsichtigem Werkstoff, z.B. Glas, besteht.

## Claims

1. An arrangement comprising a device (1) which can be used in conjunction with water in combination with at least one source (9) emitting heat rays into the region of the device (1) which can be used with water, wherein the heat ray-emitting source is a source (9) for infrared rays, wherein the source (9) is provided in a space adjoining at least one wall (23) of the device (1) which can be used with water,
**characterised in that** the infrared ray-emitting source (9) is displaceable in height and/or horizontally displaceable and/or displaceable in depth relative to a window (16) in the wall (23) between the source (9) and the device (1) which can be used with water.

2. An arrangement according to claim 1 **characterised in that** the source (9) for infrared rays is an infrared radiator.

3. An arrangement according to claim 1 or claim 2 **characterised in that** the device (1) which can be used in conjunction with water is a shower cubicle, a swimming bath, a whirlpool, a steam shower and/or a bath tub.

4. An arrangement according to one of claims 1 to 3 **characterised in that** associated with the window (16) for infrared rays to pass therethrough is a cover (8) which is infrared ray-transmissive.

5. An arrangement according to claim 4 **characterised in that in that** a device for producing a film of water is associated with the cover (8) of the window (16) for heat rays to pass therethrough, in particular infrared rays.

6. An arrangement according to one of claims 1 to 5 **characterised in that** the device (1) which is operated in conjunction with water, in particular a shower, is open upwardly.

7. An arrangement according to one of claims 1 to 6 **characterised in that** associated with the device (1) which can be used in conjunction with water, in particular a shower, is at least one light source, in particular an LED, for light therapy, and/or at least one sound source, in particular music and/or audio, for sound therapy.

8. An arrangement according to claim 7 **characterised in that** the sound source includes a structure-borne sound transducer arranged in the region of the window (16), in particular laterally beside the window (16) for heat rays to pass therethrough, in particular infrared rays.

9. An arrangement according to one of claims 7 and 8 **characterised in that** the infrared radiator and installations like sprays, nozzles, water conduits and/or devices for treating and/or heating water, for the device (1) operated with water and/or electronic components of the sound source are installed in a prefabricated component, in particular a cabinet which is associated with the device (1) operated with water.

10. An arrangement according to claim 9 **characterised in that** provided in the component, in particular the cabinet, is at least one storage means for bathing utensils.

11. An arrangement according to claim 9 or claim 10 **characterised in that** the component, in particular the cabinet, at least partially comprises transparent material, for example glass.

## Revendications

1. Agencement avec un mécanisme (1) utilisable en relation avec de l'eau, en combinaison avec au moins une source (9) émettrice de rayonnements thermiques au niveau du mécanisme (1) utilisable avec de l'eau, selon lequel la source émettrice de rayonnements thermiques est une source (9) pour des rayons infrarouges, selon lequel la source (9) est prévue dans une salle qui se trouve en bordure d'au moins une paroi (23) du mécanisme (1) utilisable avec de l'eau,
**caractérisé en ce que**
la source (9) émettrice de rayons infrarouges peut être ajustée en hauteur et/ou peut être ajustée à l'horizontale et/ou peut être ajustée dans la profondeur par rapport à une fenêtre (16) qui se trouve dans la paroi (23) située entre la source (9) et le mécanisme (1) utilisable avec de l'eau.

2. Agencement selon la revendication 1, **caractérisé en ce que** la source (9) pour rayons infrarouges est un diffuseur à infrarouge.

3. Agencement selon la revendication 1 ou 2, **caractérisé en ce que** le mécanisme (1) utilisable en relation avec de l'eau est une cabine de douche, une piscine, un jacuzzi, une douche à vapeur et/ou une baignoire.

4. Agencement selon l'une des revendications 1 à 3, **caractérisé en ce qu'**un recouvrement (8) laissant passer les rayons infrarouges est associé à la fenêtre (16) pour permettre le passage des rayons infrarouges.

5. Agencement selon la revendication 4, **caractérisé en ce qu'**un mécanisme destiné à la production d'un film aqueux est associé au recouvrement (8) de la fenêtre (16) pour permettre le passage des rayonnements thermiques, en particulier des rayons infrarouges.

6. Agencement selon l'une des revendications 1 à 5, **caractérisé en ce que** le mécanisme (1) fonctionnant en relation avec de l'eau, en particulier une cabine de douche, est ouvert vers le haut.

7. Agencement selon l'une des revendications 1 à 6, **caractérisé en ce qu'**au moins une source d'éclairage, en particulier des diodes électroluminescentes (DEL), utilisées à des fins de luminothérapie, et/ou au moins une source sonore, en particulier de la musique et/ou des pistes audio, utilisée à des fins de thérapie par le son, sont associées au mécanisme (1) pouvant être utilisé en relation avec de l'eau, en particulier une cabine de douche.

8. Agencement selon la revendication 7, **caractérisé en ce que** la source sonore comprend un transducteur acoustique, lequel est disposé au niveau de la fenêtre (16), en particulier latéralement à côté de la fenêtre (16), pour permettre le passage des rayonnements thermiques, en particulier des rayons infrarouges.

9. Agencement selon l'une des revendications 7 à 8, **caractérisé en ce que** le diffuseur à infrarouge et des installations telles que des pommeaux, des gicleurs, des canalisations d'eau et/ou des mécanismes destinés au traitement et/ou au chauffage de l'eau, pour le mécanisme (1) fonctionnant avec de l'eau et/ou les composants électroniques de la source sonore, sont mis en place dans un élément structurel préfabriqué, en particulier une armoire, lequel est associé au mécanisme (1) fonctionnant avec de l'eau.

10. Agencement selon la revendication 9, **caractérisé en ce qu'**au moins une étagère est prévue pour les accessoires de bain dans l'élément structurel, en particulier dans l'armoire.

11. Agencement selon la revendication 9 ou 10, **caractérisé en ce que** l'élément structurel, en particulier l'armoire, est constitué, tout au moins en partie, d'un matériau transparent, par exemple du verre.
